(19) Europäisches Patentamt
European Patent Office
Office européen des brevets

(11) Veröffentlichungsnummer: **0 303 571**
**A1**

(12) # EUROPÄISCHE PATENTANMELDUNG

(21) Anmeldenummer: **88810533.5**

(22) Anmeldetag: **04.08.88**

(51) Int. Cl.4: **C 07 D 498/08**
A 61 K 31/395

(30) Priorität: **13.08.87 CH 3124/87**

(43) Veröffentlichungstag der Anmeldung:
**15.02.89 Patentblatt 89/07**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Kump, Wilhelm, Dr.**
**Friedrich-Oser-Strasse 10**
**CH-4105 Biel-Benken (CH)**

**Traxler, Peter, Dr.**
**Bündtenring 5**
**CH-4124 Schönenbuch (CH)**

Patentansprüche für folgende Vertragsstaaten: ES + GR.

(54) **Biphenylyl-Verbindungen.**

(57) Beschrieben sind Rifamycinderivate der Formel

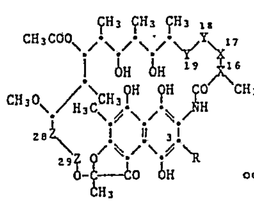

(IA)

oder

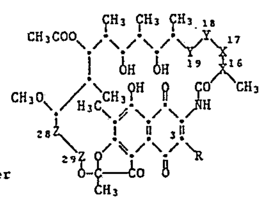

(IB)

worin R einen Rest der Formel 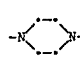$-CH_2-R_1$ (IC) darstellt,

in welchem $R_1$ für einen gegebenenfalls substituierten Biphenylylrest steht, und worin X-X, Y-Y und Z-Z je Vinylen der Formel CH=CH darstellen, oder worin X-X und Y-Y je Ethylen der Formel $CH_2$-$CH_2$ und Z-Z Ethylen oder Vinylen bedeuten, und Verfahren zu ihrer Herstellung. Diese Verbindungen können als antibakterielle und antivirale Arzneimittel verwendet werden.

EP 0 303 571 A1

## Beschreibung

### Biphenylyl-Verbindungen

Gegenstand der vorliegenden Erfindung sind neue Derivate des Rifamycins SV und S mit antibiotischer Wirksamkeit. Es handelt sich um in 3-Stellung einen 4-substituierten 1-Piperazinyl-Rest enthaltende Rifamycin-Verbindungen der Formel

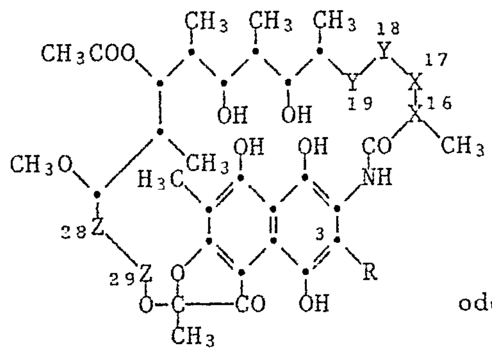

(IA)

oder

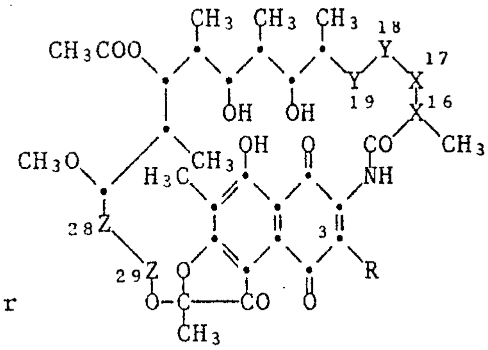

(IB)

worin R einen Rest der Formel 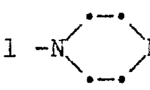$-CH_2-R_1$ (IC) darstellt,

in welchem $R_1$ für einen gegebenenfalls substituierten Biphenylylrest steht, und worin X-X, Y-Y und Z-Z je Vinylen der Formel CH=CH darstellen, oder worin X-X und Y-Y je Ethylen der Formel $CH_2$-$CH_2$ und Z-Z Ethylen oder Vinylen bedeuten, und Salze von solchen Verbindungen.

Die Erfindung betrifft ferner Verfahren zur Herstellung der Verbindungen der Formeln IA und IB, sowie ihrer Salze, diese enthaltende pharmazeutische Präparate, und die Verwendung dieser Verbindungen und Präparate.

Die in der vorliegenden Beschreibung angewandte Numerierung bezieht sich auf die z.B. im USA Patent Nr. 4 005 077 verwendete.

Infolge der engen Beziehung zwischen der 1,4-Chinon- und 1,4-Hydrochinon-Form (entsprechend Rifamycin S und SV) und der Leichtigkeit, mit welcher die beiden Formen ineinander übergehen, sind überall, wo nicht spezifisch anders angegeben, beide Formen Teil der Erfindung, wobei jedoch die SV-Form (IA) als die bevorzugte anzusehen ist.

Der gegebenenfalls substituierte Biphenylylrest $R_1$ kann ein entsprechender 3-, vorzugsweise ein 2- und in erster Linie ein 4-Biphenylylrest sein, der z.B. durch Niederalkyl, vorzugsweise mit 1 bis 4 Kohlenstoffatomen, wie Methyl, Ethyl, n-Propyl, Isopropyl, n-Butyl, Isobutyl oder tert.-Butyl, Niederalkoxy, vorzugsweise mit 1 bis 4 Kohlenstoffatomen, z.B. Methoxy, Ethoxy or Isopropyloxy, oder durch Halogen, vorzugsweise z.B. Fluor oder Chlor, ferner Brom, substituiert sein kann, wobei ein oder mehrere gleiche oder verschiedene Substituenten in einem oder beiden aromatischen Ringen der Biphenylylgruppe $R_1$ vorhanden sein können.

Sofern nicht abweichend definiert, sind unter mit "nieder" bezeichneten Resten beispielsweise solche zu verstehen, die bis und mit 7, insbesondere bis und mit 4, C-Atome aufweisen.

Salze der Verbindungen der Formeln IA und IB sind insbesondere Säureadditionssalze, inbesondere therapeutische verwendbare Säureadditionssalze; Hydrochinonverbindungen der Formel IA können auch Salze mit Basen bilden.

Diese Verbindungen der Formeln IA und IB weisen u.a. eine antibakterielle Wirkung, insbesondere gegen Mycobakterien auf, wie u.a. an Mäusen oder Ratten, die z.B. mit Mycobacterium tuberculosis infiziert wurden, gezeigt werden kann. Sie weisen in solchen Tests $ED_{50}$-Werte auf, die ungefähr denjenigen des bekannten antituberkulösen Mittels Rifampicin entsprechen.

Obwohl Rifampicin zu den bevorzugten Mitteln bei der Behandlung insbesondere von tuberkulösen Infektionen zählt, ist seine relativ kurze Verweildauer im Organismus in manchen Fällen ein wesentlicher Nachteil.

Die Bereitstellung von Wirkstoffen, die im Vergleich zu Rifampicin eine etwa gleich starke, dabei aber verlängerte Wirkung gegenüber Tuberkulose-Infektionen aufweisen, ist daher von vordringlicher Bedeutung. Die z.B. im USA Patent 4 005 077 beschriebenen 3-(4-Benzyl-1-piperazinyl)-rifamycine weisen den gewünschten Vorteil nicht auf; sie übertreffen zwar Rifampicin in Bezug auf antituberkulöse Wirkung in vivo um etwa das Dreifache, sind ihm aber in Bezug auf die Langzeitwirkung kaum überlegen.

Dagegen wurde nun gefunden, dass sich die neuen Verbindungen gemäss vorliegender Erfindung überraschenderweise nicht nur durch eine gute antituberkulöse Wirksamkeit, die etwa derjenigen des Rifampicins entspricht, sondern insbesondere durch eine wesentlich erhöhte Verweildauer im Organismus auszeichnen.

Die Verbindungen der vorliegenden Erfindung weisen zudem eine, im Vergleich mit Rifampicin überraschend hohe Wirkung gegen atypische Mycobakterien, insbesondere gegen M. avium, wie M. avium complex, gegen letztere z.B. in Dosen ab etwa 0,01 µg/ml, auf. Infektionen, die auf atypische Mycobakterien, insbesondere auf M. avium, zurückzuführen sind, wurden insbesondere bei Patienten mit dem sog. "Acquired Immune Deficiency Syndrome" (AIDS) festgestellt und bilden oft die unmittelbare Todesursache von solchen Patienten.

Die neuen Verbindungen der vorliegenden Erfindung, insbesondere diejenigen, in denen X-X und Y-Y für Ethylen und Z-Z für Ethylen oder Vinylen stehen, deren Wirksamkeit gegenüber Mycobakterien, inkl. atypischen Mycobakterien, nicht derart ausgeprägt sind, hemmen in Dosen ab etwa 0,5 µg/ml zudem die reverse Transkriptase, ein für Retroviren (oder Oncornaviren), wie RNS-Tumor- und Leukämieviren, charakteristisches Enzym. Retroviren benötigen dieses Enzym für ihren natürlichen Replikationscyclus.

Der Nachweis von Typ C-Retroviren und deren reversen Transkriptase beim Menschen erfolgte erstmals bei einer T-Zellen-Leukämie; das Virus wurde human T-cell leukaemia virus (HTLV-I) genannt. Bei weiteren T-ZellLeukämien und -Lymphomen wurden das gleiche Virus und ein ähnliches Retrovirus (HTLV-II) gefunden. Schliesslich wurde ein derartiges Virus auch im Zusammenhang mit AIDs-Erkrankungen isoliert. Dieses wurde anfänglich mit HTLV-III oder LAV bezeichnet; die heute gültige Bezeichnung für diese Gruppe von Viren lautet HIV. Diese, der HTLV-Familie angehörenden Retroviren benötigen die reverse Transkriptase für die Bildung einer Doppelstrang-DNA (Provirus), welche in das Zell-Genom "integriert" wird und zur malignen Transformation führen kann.

Während nach der Induktion maligner leukämischer, lymphatischer oder tumoröser Neubildungen durch Retroviren eine Abnahme der reversen Transkriptase und des Virus HTLV-I und -II nachgewiesen werden kann und sekundäre, durch diese Viren induzierte Oncogene die Replikation der malignen Zellen steuern, ist das Virus HIV und die reverse Transkriptase bei AIDS nicht nur im Frühstadium, sondern während des ganzen Krankheitsverlaufs nachweisbar. Die laufende Infektion und die Funktions-störung von immunkompetenten T-Helfer-Zellen führen schliesslich zum Zusammenbruch des Immunsystems. Es ist anzunehmen, dass eine Hemmung der reversen Transkriptase und damit der Virusreplikation bei positivem Enzym- und Virusantigen-Nachweis den Krankheitsprozess beeinflusst. Bei der Induktion von Leukämien und Lymphomen durch Retroviren (HTLV-I und HTLV-II), wie möglicherweise auch bei durch Retroviren induzierten Sarkomen oder Mammakarzinomen, sollte dagegen eine prophylaktische Anwendung möglich sein, sofern eine leicht und breit verfügbare diagnostische Erfassung von derartigen Risikopatienten vorausgesetzt werden kann.

Auch im Zusammenhang mit der nicht-A- und nicht-B-Hepatitis ist die reverse Transkriptase als spezifisches Enzym in Assoziation mit Viruspartikeln gefunden worden.

Die neuen Verbindungen der vorliegenden Erfindung weisen zudem gute antibakterielle Eigenschaften gegen andere, insbesondere gram-positive Mikroorganismen auf. So zeigen sie im in vitro-Versuch gegenüber Staphylokokken, wie Staphylococcus aureus K 1098, und gegenüber Streptokokken, wie Streptococcus pyogenes Aronson K 1129, Hemmwirkungen ab etwa 0,005 µg/ml. Im in vivo-Versuch, z.B. gegen den obgenannten Staphylococcus, sind die Verbindungen der vorliegenden Erfindung in Dosen ($ED_{50}$) ab etwa 1 mg/kg sowohl bei subkutaner, wie auch oraler Verabreichung wirksam.

Dabei weisen die Verbindungen gemäss der vorliegenden Erfindung eine grosse therapeutische Breite auf, indem sie eine signifikante Toxizität erst bei sehr hohen Dosen, etwa in der Grössenordnung von 5000 mg/ka, erreichen.

Die neuen Verbindungen können daher als Heilmittel, in erster Linie zur Behandlung von bakteriellen, üblicherweise tuberkulösen Erkrankungen, insbesondere von solchen, die durch atypische Mycobakterien ausgelöst werden, und von viralen Erkrankungen, insbesondere von solchen, die durch Typ-C-Retroviren, wie Viren vom Typ HTLV und HIV, ausgelöst werden, insbesondere bei der Behandlung von AIDS-Erkrankten verwendet werden, wobei sie prophylaktisch oder therapeutisch eingesetzt werden können. Dabei steht die Behandlung mit Verbindungen der vorliegenden Erfindung von durch atypische Mycobakterien, insbesondere M. avium, und durch Typ-C-Retroviren verursachten oder mitverursachten malignen Erkrankungen, aber auch von gewissen Immunkrankheiten und Autoimmunkrankheiten im Vordergrund. Als Tumorkrankheiten sind in erster Linie durch Retroviren verursachte Leukämien, Lymphome und Lymphosarkome, sowie möglicherweise auch Osteosarkome und Mammacarcinome zu nennen, wobei sich die Verbindungen der vorliegenden Erfindung auch zur Rezidivprophylaxe nach chirurgischer Therapie, Strahlentherapie oder zytostatischer oder antimetabolischer Chemotherapie eignen. Als Immunkrankheit sei AIDS, als Autoimmunkrankheit systemischer Lupus erythematosus genannt, bei denen nach neueren Befunden RNS-Tumorviren auch eine wichtige Rolle zu spielen scheinen.

Die erfindungsgemässen Verbindungen können ebenfalls als Ausgangsmaterialien für die Herstellung wertvoller Pharmazeutika verwendet werden.

Die Erfindung betrifft insbesondere Verbindungen der Formeln IA und IB, worin R für einen Rest der Formel IC steht, in welcher $R_1$ gegebenenfalls durch Niederalkyl, Niederalkoxy und/oder Halogen substituiertes Biphenylyl darstellt, worin Niederalkyl und Niederalkoxy vorzugsweise bis und mit 4 Kohlenstoffatome enthalten und in erster Linie Methyl bzw. Methoxy bedeuten, und Halogen insbesondere für Fluor oder Chlor

steht, und worin Biphenylyl vorzugsweise 2-Biphenylyl und in erster Linie 4-Biphenylyl darstellt, und worin X-X, Y-Y und Z-Z die oben gegebenen Bedeutungen haben, sowie Salze, insbesondere pharmazeutisch verwendbare Salze von solchen Verbindungen.

Die Erfindung betrifft vorzugsweise Verbindungen der Formel IA und IB, worin R für einen Rest der Formel IC steht, in welcher $R_1$ für gegebenenfalls durch Niederalkyl mit bis und mit 4 Kohlenstoffatomen, besonders Methyl, und/oder Halogen, besonders Fluor oder Chlor, substituiertes Biphenylyl, insbesondere 2-Biphenylyl und in erster Linie 4-Biphenylyl steht, und worin X-X, Y-Y und Z-Z für Vinylen, oder X-X und Y-Y für Ethylen und Z-Z für Vinylen oder Ethylen stehen, sowie Salze, insbesondere pharmazeutisch verwendbare Salze von solchen Verbindungen.

Die Erfindung betrifft in erster Linie Verbindungen der Formel IB, und vorzugsweise der Formel IA, worin R für einen Rest der Formel IC steht, in welcher $R_1$ für durch Methyl substituiertes, insbesondere jedoch für unsubstituiertes 4-Biphenylyl steht, und worin X-X, Y-Y und Z-Z Vinylen oder X-X und Y-Y Ethylen und Z-Z Vinylen oder Ethylen bedeuten, sowie Salze, insbesondere pharmazeutisch verwendbare Salze von solchen Verbindungen.

Die Erfindung betrifft vor allem das 3-[4-(4-Biphenylyl-methyl)-1-piperazinyl]-rifamycin SV, sowie das entsprechende 3-[4-(4-Biphenylyl-methyl)-1-piperazinyl]-16,17,18,19-tetrahydro-rifamycin SV und -16,17,18,19,28,29-hexahydro-rifamycin SV, und Salze, insbesondere die pharmazeutisch verwendbaren Salze von solchen Verbindungen.

Die neuen erfindungsgemässen Verbindungen der Formeln IA und IB und können mittels an sich bekannter allgemeiner Analogieverfahren hergestellt werden, z.B. indem man

(a) ein 3-$R_o^1$-Rifamycin S oder 3- $R_o^1$-Rifamycin SV oder eine entsprechende 16,17,18,19-Tetrahydro- oder 16,17,18,19,28,29-Hexahydro-Verbindung, worin $R_o^1$ für N-Piperazinyl steht, mit einem, den Rest der Formel $-CH_2-R_1$ (IIIA) einführenden Mittel umsetzt, oder

(b) zur Herstellung einer Verbindung der Formel (IB) ein 3-$R_o^2$-Rifamycin S oder ein 16,17,18,19-Tetrahydro- oder 16,17,18,19,28,29-Hexahydroderivat davon, worin $R_o^2$ für Wasserstoff oder Halogen steht, mit einer Verbindung der Formel

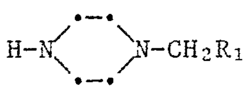

(II)

umsetzt, und, wenn erwünscht, eine verfahrensgemäss erhältliche Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

In der Verfahrensvariante a) verwendet man als Reagens zur Einführung des Restes der Formel IIIA reaktionsfähige Ester des entsprechenden Alkohols, vor allem Verbindungen der Formel $X-CH_2-R_1$ (III), worin X für den Rest einer starken anorganischen oder organischen Säure steht, wie für den Rest einer Halogen-, wie Chlor-, Brom- oder Iodwasserstoffsäure, einer sauerstoffhaltigen anorganischen Säure, wie Schwefelsäure, Phosphorsäure, phosphorige Säure, Kieselsäure oder schweflige Säure, oder einer Halogenschwefelsäure, wie Fluorsulfonsäure, oder einer organischen Sulfonsäure, wie einer aliphatischen oder aromatischen Sulfonsäure, z.B. einer Niederalkansulfonsäure, wie Methansulfonsäure, oder einer gegebenenfalls, z.B. durch Niederalkyl oder Nitro, substituierten Benzol sulfonsäure, wie p-Toluolsulfonäsure oder 4-Nitrobenzolsulfonsäure steht. X bedeutet insbesondere Chlor, Brom oder Iod, ferner Methansulfonyloxy oder p-Toluolsulfonyloxy.

Die Umsetzung erfolgt vorzugsweise in Anwesenheit einer Base, insbesondere eines stark basischen, nicht nucleophilen, tertiären Amins, vorzugsweise eines entsprechenden sterisch gehinderten aliphatischen und/oder araliphatischen Amins, wie Tri-niederalkyl-amins, z.B. der sogenannten Hünig-Base, d.h. Ethyl-diisopropylamins. Dabei werden das Rifamycin-Ausgangsmaterial und das Alkylierungsmittel in etwa äquimolaren Mengen verwendet, wobei die Base vorzugsweise ebenfalls im äquimolaren Verhältnis zugesetzt wird.

Dabei arbeitet man vorzugsweise in Gegenwart eines geeigneten Lösungsmittels oder eines Lösungsmittelgemisches, und, wenn notwendig, unter Kühlen oder Erwärmen, z.B. in einem Temperaturbereich von etwa 0°C bis etwa +100°C, und/oder in der Atmosphäre eines Inertgases.

Die Verfahrensvariante b) wird in an sich bekannter Weise durchgeführt. So verwendet man bei der Reaktion mit Ausgangsstoffen vom 3-$R_o^2$-Rifamycin-Typ S, worin $R_o^2$ für Wasserstoff oder Halogen steht, zweckmässig einen Ueberschuss , z.B. etwa einen 5- bis 10-fachen Ueberschuss, einer Verbindung der Formel II. Die Reaktion wird z.B. in einem Hydroxygruppen-freien organischen Lösungsmittel, vorzugsweise von geringer Polarität, wie einem halogenierten aliphatischen Kohlenwasserstoff, wie Methylenchlorid oder Chloroform, einem Ester oder Ether, wie z.B. Ethylacetat, Butylacetat, Amylacetat, 1,2-Dimethoxyethan oder Tetrahydrofuran, und in erster Linie in Dioxan, oder in einem Lösungsmittelgemisch und vorzugsweise bei Zimmertemperatur oder, z.B. bei zu langsamem Verlauf, bei erhöhter Temperatur, z.B. bis zu 100°, wenn notwendig, in der Atmosphäre eines inerten Gases durchgeführt. Dabei kann der Verlauf der Reaktion

dünnschichtchromatographisch verfolgt werden.

Im allgemeinen bildet sich bei dieser Verfahrensvariante ein Gemisch des erwünschten Reaktionsproduktes in der Chinon- (Formel IB) und der Hydrochinonform (Formel IA). Vorzugsweise wird dieses Gemisch, wie unten noch näher beschrieben, vereinheitlicht, wobei mittels Reduktion die Hydrochinonform (Derivat der SV-Reihe) und mittels Oxydation die Chinon-form (Derivat der S-Reihe) gebildet werden kann.

In 3-$R_o^2$-Rifamycin S-Ausgangsstoffen, in denen $R_o^2$ Halogen ist, bedeutet $R_o^2$ ausser z.B. Chlor und Iod in erster Linie Brom. Die Reaktion mit einer Verbindung der Formel II wird üblicherweise in einem inerten Lösungsmittel, insbesondere in einem Ether, wie Tetrahydrofuran oder Dioxan, oder in einem halogenierten aliphatischen Kohlenwasserstoff, wie Chloroform, Dichlormethan oder 1,2-Dichlorethan, oder in einem aromatischen Kohlenwasserstoff, wie Benzol oder Toluol, oder einem Gemisch vorgenommen, wobei die Reaktion, wenn erforderlich unter Kühlen oder Erwärmen, vorzugsweise bei Temperaturen zwischen 0°C bis 100°C, und, wenn notwendig, in der Atmosphäre eines Inertgases, durchgeführt wird.

Die Umwandlung von Verbindungen der Formeln IA und IB in andere Verbindungen der Formeln IA und/oder IB kann in an sich bekannter Weise erfolgen.

So kann man z.B. Verbindungen der Formeln IA und IB, worin X-X und Y-Y für Ethylen und Z-Z für Ethylen oder Vinylen stehen, erhalten, indem man in einer Verbindung der Formel IA oder IB, worin X-X, Y-Y und Z-Z für Vinylen oder X-X und Y-Y für Ethylen und Z-Z für Vinylen stehen, die Doppelbindungen der Vinylengruppen sättigt.

Die Sättigung der Doppelbindungen erfolgt in an sich bekannter Weise, üblicherweise mittels katalytischer Hydrierung. Dabei arbeitet man mit Wasserstoff bei normalem oder erhöhtem Druck unter den Bedingungen der heterogenen oder homogenen Katalyse. Als Katalysatoren für erstere kommen Metallkatalysatoren, z.B. Raney-Metalle, wie Raney-Nickel, oder Edelmetallkatalysatoren, wie Palladium, Platin, Platinoxid oder Rhodium, welche gegebenenfalls auf einem Träger, wie Calciumcarbonat oder Bariumsulphat, adsorbiert sein können, in Frage. Für die homogene Katalyse verwendet man insbesondere komplexe Rhodiumverbindungen, z.B. Tris(triphenylphosphin)-rhodium(I)chlorid.

Die Bedingungen der Hydrierung kann man so modifizieren, dass die weniger reaktionsfähige, isolierte 28,29-Doppelbindung nicht mitreduziert wird, indem man z.B. die Hydrierung beim Verbrauch von zwei Aequivalenten Wasserstoff unterbricht, und das resultierende 16,17,18,19-Tetrahydroderivat isoliert. Zu diesem Zweck verwendet man einen milderen Katalysator, wie z.B. Palladium auf einem Träger, z.B. Aktivkohle oder Calciumcarbonat, wobei die Reaktion unter Normaldruck und Raumtemperatur spontan beim Verbrauch von zwei Aequivalenten zum Stillstand kommt. Bei Anwendung von stärkeren Katalysatoren, z.B. Platin, insbesondere in der in situ aus Platinoxid durch Reduktion erhältlichen Form, kann die Hydrierung zur Sättigung aller drei Doppelbindungen führen; unter den üblichen Bedingungen kommt es zum spontanen Stillstand der Hydrierung und zur Bildung des entsprechenden 16,l7,18,19,28,29-Hexahydroderivats.

Dabei führt die Hydrierung zur Entstehung eines Asymmetriezentrums am Kohlenstoffatom 16 und somit eines Gemisches von Epimeren, die sich durch die sterische Anordnung der an das Kohlenstoffatom 16 gebundenen Methylgruppe voneinander unterscheiden. Wegen der schwierigen und zudem verlustreichen Trennung der Epimeren durch physikalische Methoden, wird üblicherweise das erhaltene Epimerengemisch als einheitliches Verfahrensprodukt isoliert und verwendet.

Die Verfahrensprodukte können sowohl in der Hydrochinonform der Formel IA, als auch in der Chinonform der Formel IB oder, insbesondere das Produkt der Verfahrensmodifikation b), in Form eines Gemisches der beiden Formen erhalten werden; man kann sie nachträglich in an sich bekannter Weise ineinander bzw. ein Gemisch der beiden in eine der beiden einheitlichen Formen überführen. Dabei kann die Umwandlung mittels Reduktion bzw. Oxidation nach oder vorteilhafterweise vor der Isolierung des gewünschten produkts durchgeführt werden. Die Reduktion eines Chinons zum entsprechenden Hydrochinon kann durch Behandeln mit einem geeigneten Reduktionsmittel, wie einem Alkalimetall-, z.B. Natrium-dithionit oder -hydrosulfit, mit Zink und Essigsäure, oder vorzugsweise mit Ascorbinsäure, die Oxidation eines Hydrochinons zum entsprechenden Chinon durch Behandeln mit einem geeigneten Oxidationsmittel, wie Luftsauerstoff, Wasserstoffperoxid, Alkalimetall-, z.B. Kalium-, -ferricyanid, einem Persulfat, z.B. Ammoniumpersulfat, oder Mangandioxid bewirkt werden, wobei die Oxidation vorzugsweise unter basischen Bedingungen durchgeführt wird. Die Chinone sind meist violettrot bis schwarz gefärbte Verbindungen, während die Hydrochinone üblicherweise hell, z.B. gelb, gefärbt sind und sich üblicherweise besser kristallisieren lassen.

Die Isolierung der Reaktionsprodukte aus einem verfahrensgemäss erhältlichen Reaktionsgemisch erfolgt in an sich bekannter Weise, z.B. durch Verdünnen mit Wasser, und/oder gegebenenfalls durch Neutralisieren mit einer wässrigen Säure, wie einer anorganischen oder organischen Säure, z.B. einer Mineralsäure oder Zitronensäure, und Zugabe eines mit Wasser nicht mischbaren Lösungsmittels, wie z.B. eines chlorierten Kohlenwasserstoffs, z.B. Chloroform oder Methylenchlorid, wobei das Reaktionsprodukt in die oranische Phase übergeht, aus welcher es in üblicher Weise, z.B. nach Trocknen durch Eindampfen des Lösungsmittels und Kristallisation und/oder Chromatographie des Rückstandes oder andere übliche Reinigungsmethoden in reiner Form erhalten werden kann.

Die Verbindungen der vorliegenden Erfindung können Salze, insbesondere Säureadditionssalze und in erster Linie pharmazeutisch verwendbare Säureadditionssalze mit anorganischen oder organischen Säuren bilden. Solche sind u.a. Halogen-, z.B. Chlor- und Bromwasserstoffsäure, Schwefelsäure, Phosphorsäure, Salpetersäure oder Perchlorsäure, oder aliphatische, alicyclische, aromatische oder heterocyclische Carbon-oder Sulfonsäuren, wie Ameisen-, Essig-, Propion-, Bernstein-, Glykol-, Milch-, Aepfel-, Wein-,

Zitronen-, Fumar-, Malein-, Hydroxymalein-, Oxal-, Brenztrauben-, Phenylessig-, Benzoe-, p-Aminobenzol-, Anthranil-, p-Hydroxybenzoe-, Salicyl-, p-Aminosalicyl-, Embon-, Methansulfon-, Ethansulfon-, Hydroxyethansulfon-, Ethylendisulfon-, Halogenbenzolsulfon-, Toluolsulfon-, Naphthalensulfon- oder Sulfanilsäuren, ferner Methionin, Tryptophan, Lysin oder Arginin, sowie Ascorbinsäure. Hydrochinonverbindungen vom Typ der Formel IA können auch Salze mit Basen, z.B. Alkalimetall-, wie Natrium- oder Kaliumsalze bilden.

Die Salzbildung, sowie das Freisetzen der Verbindungen der Formeln IA und IB aus ihren Salzen erfolgt in an sich bekannter Weise. So können Hydrochinone der Formel IA in entsprechende Salze mit Basen, vor allem in Alkalimetallsalze, durch Behandeln mit einer entspechenden Base, insbesondere einem geeigneten Alkalimetallhydroxid, -carbonat oder -hydrogencarbonat übergeführt werden; die entsprechenden Salze können durch Ansäuern, z.B. mit anorganischen Säuren, wie z.B. einer Halogenwasserstoffsäure, in die freien Hydrochinon-Verbindungen umgewandelt werden. Basische Endstoffe, können z.B. durch Behandeln mit zur Salzbildung geeigneten Säuren, wie einer der obgenannten, in ihre Säureadditionssalze umgewandelt werden; durch Behandeln mit basischen Mitteln, wie anorganischen Hydroxiden, Carbonaten und Hydrogencarbonaten, oder organischen Basen oder Ionenaustauschern kann man aus den Säureadditionssalzen die freien Verbindungen erhalten.

Verbindungen der vorliegenden Erfindung können auch innere Salze bilden, die man z.B. durch übliches Titrieren zum Neutralpunkt bzw. zum isoelektrischen Punkt erhalten kann.

Diese oder andere Salze, wie z.B. die Pikrate, können auch zur Reinigung der erhaltenen Verbindungen dienen, indem man die freien Verbindungen in Salze überführt, diese abtrennt und aus den Salzen wiederum die freien Verbindungen gewinnt. Infolge der engen Beziehung zwischen den Verbindungen in freier Form und in Form ihrer Salze sind im vorausgegangenen und nachfolgend unter den freien Verbindungen sinn- und zweckgemäss gegebenenfalls auch die entsprechenden Salze zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen.

Die im obigen Verfahren verwendeten Ausgangsstoffe sind bekannt oder können in an sich bekannter Weise hergestellt werden.

So kann man die Ausgangsstoffe für die Verfahrensvariante a) durch Behandeln von Rifamycin S mit Piperazin, in welchem eines der Ringstickstoffatome durch eine leicht abspaltbare Schutzgruppe, wie einen hydrolytisch abspaltbaren Acylrest, z.B. Niederalkoxycarbonyl, wie Methoxycarbonyl oder Aethoxycarbonyl, oder einen hydrogenolytisch abspaltbaren Benzylrest geschützt sein kann, und, wenn notwendig, durch nachfolgendes Abspalten einer vorhandenen Schutzgruppe im Piperazinrest mittels, z.B. basischer, Hydrolyse oder Hydrogenolyse, z.B. mit Wasserstoff in Gegenwart eines Hydrierungskatalysators, erhalten; dabei kann man z.B. nach dem oben für die Variante b) beschriebenen Verfahren vorgehen. Eine so erhältliche 3-$R_o^1$-Rifamycin S-Verbindung kann nach dem oben beschriebenen Verfahren mittels Reduktion, z.B. durch Behandeln mit Ascorbinsäure, in die entsprechende 3-$R_o^1$-Rifamycin SV-Verbindung, eine erhältliche 3-$R_o^1$-Rifamycin SV-Verbindung mittels Oxidation, z.B. Behandeln mit Mangandioxid, in die entsprechende 3-$R_o^1$-Rifamycin S-Verbindung, bzw. ein erhaltenes Gemisch der beiden Formen entsprechend in die eine oder die andere Form übergeführt werden. Vom entsprechenden 16,17,18,19-Tetrahydro- oder 16,17,18,19,28,29-Hexahydro-Rifamycin S ausgehend, das man z.B. durch Hydrierung, z.B. nach dem oben beschriebenen Verfahren, und Umwandeln einer Rifamycin SV-Verbindung in die entsprechende Rifamycin S-Verbindung erhalten kann, kann man die entsprechenden 16,17,18,19-Tetrahydro-, bzw. 16,17,18,19,28,29-Hexahydro-Ausgangsstoffe erhalten.

Die Ausgangsstoffe der Verfahensvariante b) sind teilweise bekannt; die 16,17,18,19-Tetrahydro- und 16,17,18,19,28,29-Hexahydro-Verbindungen kann man mittels Reduktion, z.B. katalytischer Hydrierung nach dem oben beschriebenen Verfahren, erhalten, wobei man verfahrensgemäss erhältliche Rifamycin Sv-Verbindungen, wie oben beschrieben in die entsprechenden Rifamycin S-Verbindungen und umgekehrt überführen kann.

Die vorliegende Erfindung umfasst ebenfalls die Verwendung der erfindungsgemässen Verbindungen der Formeln IA und IB und deren pharmazeutisch verwendbaren Salze, gegebenenfalls zusammen mit Hilfsstoffen und/oder in Kombination mit anderen Wirkstoffen, insbesondere Antibiotika oder Chemotherapeutika, zur Herstellung entsprechender Arzneimittel bzw. als Mittel zur Behandlung von Krankheiten, wie den oben beschriebenen, wobei sie sowohl prophylaktisch, als auch kurativ eingesetzt werden können. Die erfindungsgemässen Wirkstoffe werden in wirksamen Mengen, vorzugsweise in Form von pharmazeutischen Zusammensetzungen zusammen mit konventionellen pharmazeutischen Trägermaterialien oder Hilfsstoffen, verabreicht. Dabei werden z.B. an Warmblüter mit einem Körpergewicht von etwa 70 kg je nach Spezies, Alter und individuellem Zustand, sowie je nach Applikationsweise und insbesondere auch je nach der besonderen Wirkung auf den jeweiligen Krankheitserreger tägliche Dosen von etwa 50 bis etwa 3000 mg, z.B. 600 mg, die in akuten Fällen noch überschritten werden können, eingesetzt. Sinngemäss umfasst die Erfindung auch die entsprechende Methode zur medizinischen Behandlung.

Die Erfindung betrifft im weiteren pharmazeutische Zusammensetzungen, welche die Verbindungen der vorliegenden Erfindung als Wirkstoffe enthalten, sowie Verfahren zur Herstellung dieser Zusammensetzungen.

Bei den erfindungsgemässen pharmazeutischen Zusammensetzungen handelt es sich z.B. um solche zur enteralen, wie peroralen oder rektalen, sowie zur parenteralen Verabreichung. Entsprechende Dosiseinheitsformen, insbesondere zur peroralen Verabreichung, z.B. Dragées, Tabletten oder Kapseln, enthalten vorzugsweise von etwa 50 bis etwa 500 mg, insbesondere von etwa 100 bis etwa 300 mg des Wirkstoffs, üblicherweise zusammen mit pharmazeutisch verwendbaren Träger- oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister (unter Verwendung z.B. von Mais-, Weizen-, Reis- oder Kartoffelstärke), Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykole und/oder Titandioxid enthalten, oder Lacklösungen in geeigneten organischen Lösungsmitteln oder Lösungsmittelgemischen, oder, zur Herstellung Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmzeutische Präparate sind Steckkapseln aus Gelatine, sowie weiche geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen einer in Wasser löslichen Form des Wirkstoffes, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Zusammensetzungen der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1:

Eine Lösung von 3 g 3-Brom-rifamycin S in 50 ml Tetrahydrofuran wird mit 17,5 g 1-(4-Biphenylyl-methyl)-piperazin versetzt und bei 20° während 30 Minuten stehen gelassen. Anschliessend säuert man durch Zugabe von wässriger Zitronensäurelösung an und nimmt das Reaktionsprodukt in Methylenchlorid auf. Nach dem Trocknen und Eindampfen des organischen Extraktes hinterbleibt ein dunkelgefärbter Rückstand, das 3-[4-(4-Biphenylyl-methyl)-l-piperazinyl]-rifamycin S der Summenformel $C_{54}H_{63}N_3O_{12}$. Man löst diesen Rückstand in Methanol und setzt tropfenweise wässrige Ascorbinsäure zu. Das 3-[4-(4-Biphenylyl-methyl)-l-piperazinyl]-rifamycin SV fällt in kristalliner Form aus, Smp. 172-174°; Massenspektrum $(MS:(M+1)^+)$ = m/e 948, entsprechend der Summenformel $C_{54}H_{65}N_3O_{12}$.

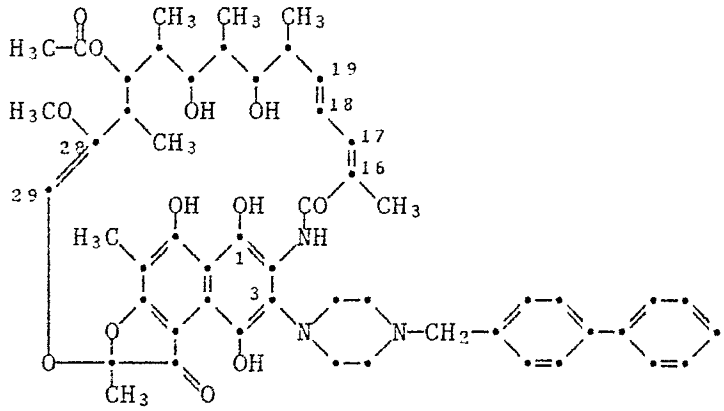

Beispiel 2:

Das Gemisch von 2 g Rifamycin S und 2 g 1-(4-Biphenylyl-methyl)-piperazin in 50 ml Dixoan wird während 24 Stunden bei Raumtemperatur stehen gelassen und dann analog dem Verfahren des Beispiels 1, inkl. die Behandlung mit wässriger Ascorbinsäure, aufgearbeitet. Man erhält so das 3-[4-(4-Biphenylyl-methyl)-1-piperazinyl]-rifamycin SV in kristalliner Form, Smp. 172-174°.

Zur Herstellung des Natriumsalzes werden äquivalente Mengen 3-[4-(4-Biphenylyl-methyl)-1-piperazinyl]-rifamycin SV und Natriumhydrogencarbonat in einem gemisch von Dioxan und Wasser gelöst und die Lösung lyophilisiert.

Beispiel 3:

Eine Lösung von 2 g 3-[4-(4-Biphenylyl-methyl)-1-piperazinyl]-rifamycin SV in 100 ml Ethanol wird in Gegenwart von 0,2 g 10%igem (G/G) Palladium-auf-Kohle während 5 Std. bei Raumtemperatur und unter Normaldruck hydriert. Der Katalysator wird durch eine Schicht von Kieselgur abfiltriert, das Filtrat zur Trockne eingedampft und der Rückstand aus einem Gemisch von Ethylacetat und Diethylether kristallisiert. Man erhält so das 3-[4-(4-Biphenyly-methyl)-1-piperazinyl]-16,17,18,19-tetrahydro-rifamycin SV (Epimerengemisch) erhalten; das Massenspektrum: $m/z = 952$ $(M+1)^+$ entspricht der Summenformel $C_{54}H_{69}N_3O_{12}$.

Beispiel 4:

Eine Lösung von 2 g 3-[4-(4-Biphenylyl-methyl)-1-piperazinyl]-rifamycin SV in 100 ml Ethanol wird in Gegenwart von 0,2 g Platinoxid während 4 Std. bei Raumtemperatur und unter Normaldruck hydriert. Die Aufarbeitung des Reaktionsgemisches analog zu Beispiel 1 ergibt das 3-[4-(4-Biphenylyl-methyl)-1-piperazinyl]-16,17,18,19,28,29-hexahydro-rifamycin SV (Epimerengemisch), dessen Massenspektrum ($m/z = 954$ $(M+l)^+$) der Summenformel $C_{54}H_{71}N_3O_{12}$ entspricht.

Beispiel 5:

Analog Beispiel 2 erhält man 3-[4-(4-Bisphenylyl-methyl)-1-piperazinyl]-16,17,18,19-tetrahydro-rifamycin SV aus 16,17,18,19-Tetrahydro-rifamycin S und 1-(4-Biphenylyl-methyl)-piperazin.

Beispiel 6:

Kapseln, enthaltend 250 mg 3-[4-(4-Biphenylyl-methyl)-1-piperazinyl]-rifamycin SV, können wie folgt hergestellt werden:

Zusammensetzung (für 1000 Kapseln):
3-[4-(4-Biphenylyl-methyl)-1-piperazinyl]-
rifamycin SV 250,0 g
Maisstärke 50,0 g
Polyvinylpyrrolidon 15,0 g
Magnesiumstearat 5,0 g
Aethanol q.s.

Der Wirkstoff und die Maisstärke werden vermischt und mit einer Lösung des Polyvinylpyrrolidons in 50 g

Aethanol befeuchtet. Die feuchte Masse wird durch ein Sieb mit einer Maschenweite von 3 mm gepresst und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

**Patentansprüche**

1. Rifamycinderivate der Formel

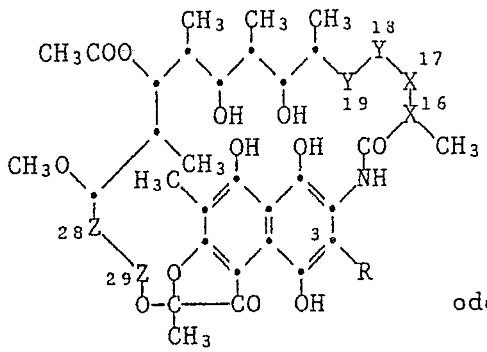

(IA)

oder

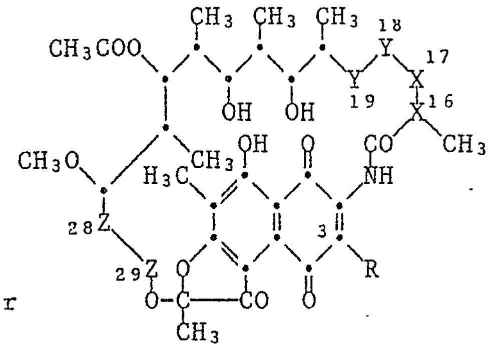

(IB)

worin R einen Rest der Formel 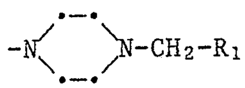 (IC) darstellt,

in welchem $R_1$ für einen gegebenenfalls substituierten Biphenylylrest steht, und worin X-X, Y-Y und Z-Z je Vinylen der Formel CH=CH darstellen, oder worin X-X und Y-Y je Ethylen der Formel $CH_2$-$CH_2$ und Z-Z Ethylen oder Vinylen bedeuten, und Salze von solchen Verbindungen.

2. Eine Verbindung nach Anspruch 1, worin $R_1$ ein unsubstituierter oder durch Niederalkyol, Niederalkoxy oder Halogen substituierter Biphenylylrest ist, oder ein Salz davon.

3. Eine Verbindung nach Anspruch 2, worin $R_1$ ein unsubstituierter oder durch Niederalkyl bis und mit 4 C-Atomen, Niederalkoxy bis und mit 4 C-Atomen oder Halogen, insbesondere Methyl, Methoxy, Fluor oder Chlor, substituierter 2- oder 4-Biphenylylrest ist, oder ein Salz davon.

4. Eine Verbindung nach einem der Ansprüche 1-3, worin $R_1$ unsubstituiertes 4-Biphenyly ist, oder ein Salz davon.

5. Eine Verbindung der Formel IA nach einem der Ansprüche 1-4 oder ein Salz davon.

6. Eine Verbindung nach einem der Ansprüche 1-5, worin X-X, Y-Y und Z-Z je Vinylen darstellen, oder ein pharmazeutisch verwendbares Salz davon.

7. Eine Verbindung nach einem der Ansprüche 1-5, worin X-X und Y-Y je Ethylen und Z-Z Vinylen bedeuten, oder ein pharmazeutisch verwendbares Salz davon.

8. Eine Verbindung nach einem der Ansprüche 1-5, worin X-X, Y-Y und Z-Z je Ethylen bedeuten, oder ein pharmazeutisch verwendbares Salz davon.

9. 3-[4-(4-Biphenylyl-methyl)1-piperazinyl]-rifamycin SV oder ein pharmazeutisch anwendbares Salz davon nach Anspruch 1.

10. 3-[4-(4-Biphenylyl-methyl)-1-piperazinyl]-16,17,18,19-tetrahydro-rifamycin SV oder ein pharmazeutisch anwendbares Salz davon nach Anspruch 1.

11. 3-[4-(4-Biphenylyl-methyl)-1-piperazinyl]-16,17,18,19,28,29-hexahydro-rifamycin SV oder ein pharmazeutisch anwendbares Salz davon nach Anspruch 1.

12. Ein Rifamycinderivat nach einem der Ansprüche 1-11 oder ein pharmazeutisch verwendbares Salz davon zur Anwendung in einem Verfahren zur therapeutischen Behandlung des menschlichen oder tierischen Körpers.

13. Ein pharmazeutisches Präparat, welches ein Rifamycin nach einem der Ansprüche 1-11 oder ein pharmazeutisch verwendbares Salz davon zusammen mit pharmazeutischem Trägermaterial enthält.

14. Verwendung eines Rifamycinderivats nach einem der Ansprüche 1-11 zur Herstellung von Arzneimitteln, die für die Anwendung zur Behandlung bakterieller Erkrankungen bestimmt sind.

15. Verwendung eines Rifamycinderivats nach einem der Ansprüche 1-11 zur Herstellung von

Arzneimitteln, die für die Anwendung zur Behandlung viraler Erkrankungen durch Typ C-Retroviren bestimmt sind.

16. Verfahren zur Herstellung eines Rifamycinderivats der Formel

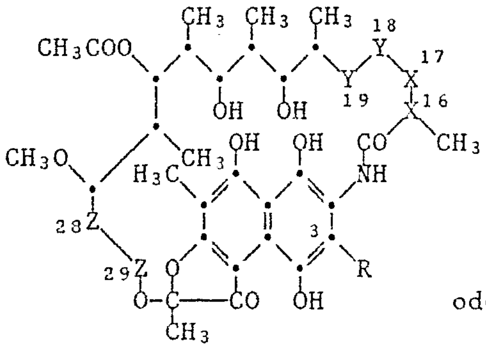

(IA)

oder

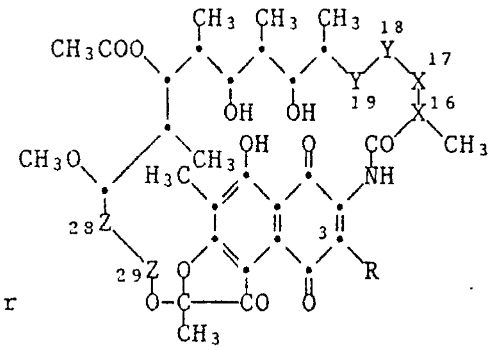

(IB)

worin R einen Rest der Formel 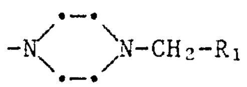 (IC) darstellt,

in welchem $R_1$ für einen gegebenenfalls substituierten Biphenylylrest steht, und worin X-X, Y-Y und Z-Z je Vinylen der Formel CH=CH darstellen, oder worin X-X und Y-Y je Ethylen der Formel $CH_2$-$CH_2$ und Z-Z Ethylen oder Vinylen bedeuten, oder eines Salzes davon, dadurch gekennzeichnet, dass man

(a) ein 3-$R_o^1$-Rifamycin S oder 3- $R_o^1$-Rifamycin SV oder eine entsprechende 16,17,18,19-Tetrahydro- oder 16,17,18,19,28,29-Hexahydro-Verbindung, worin $R_o^1$ für N-Piperazinyl steht, mit einem, den Rest der Formel -$CH_2$-$R_1$ (IIIA) einführenden Mittel umsetzt, oder

(b) zur Herstellung einer Verbindung der Formel (IB) ein 3-$R_o^2$-Rifamycin S oder ein 16,17,18,19-Tetrahydro- oder 16,17,18,19,28,29-Hexahydroderivat davon, worin $R_o^2$ für Wasserstoff oder Halogen steht, mit einer Verbindung der Formel

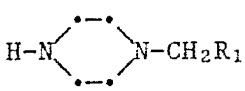

(II)

umsetzt, und, wenn erwünscht, eine verfahrensgemäss erhältliche Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

**Patentanspruche für folgenden Vertragsstaaten: ES und GR**

1. Verfahren zur Herstellung eines Rifamycinderivats der Formel

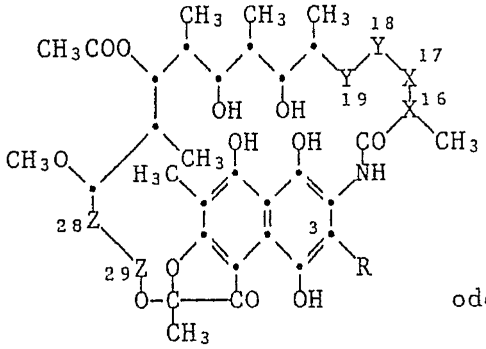

(IA)

oder

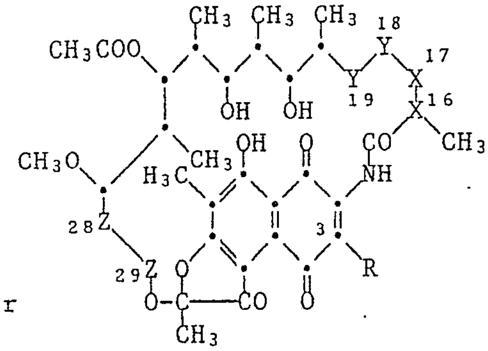

(IB)

worin R einen Rest der Formel 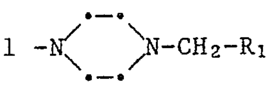 (IC) darstellt,

in welchem $R_1$ für einen gegebenenfalls substituierten Biphenylylrest steht, und worin X-X, Y-Y und Z-Z je Vinylen der Formel CH=CH darstellen, oder worin X-X und Y-Y je Ethylen der Formel $CH_2$-$CH_2$ und Z-Z Ethylen oder Vinylen bedeuten, oder eines Salzes davon, dadurch gekennzeichnet, dass man

(a) ein 3-$R_o^1$-Rifamycin S oder 3- $R_o^1$-Rifamycin SV oder eine entsprechende 16,17,18,19-Tetrahydro- oder 16,17,18,19,28,29-Hexahydro-Verbindung, worin $R_o^1$ für N-Piperazinyl steht, mit einem, den Rest der Formel -$CH_2$-$R_1$ (IIIA) einführenden Mittel umsetzt, oder

(b) zur Herstellung einer Verbindung der Formel (IB) ein 3-$R_o^2$-Rifamycin S oder ein 16,17,18,19-Tetrahydro- oder 16,17,18,19,28,29-Hexahydroderivat davon, worin $R_o^2$ für Wasserstoff oder Halogen steht, mit einer Verbindung der Formel

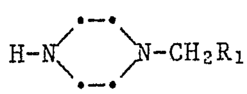 (II)

umsetzt, und, wenn erwünscht, eine verfahrensgemäss erhältliche Verbindung der Formel (IA) und/oder (IB) in eine andere Verbindung der Formel (IA) und/oder (IB) überführt, und/oder ein verfahrensgemäss erhältliches Salz in die freie Verbindung oder in ein anderes Salz und/oder eine verfahrensgemäss erhältliche freie Verbindung in ein Salz überführt.

2. Verfahren nach Anspruch 1 zur Herstellung von Verbindungen der Formel IA oder IB, worin $R_1$ ein unsubstituierter oder durch Niederalkyl, Niederalkoxy oder Halogen substituierter Biphenylylrest ist, oder eines Salzes davon.

3. Verfahren nach Anspruch 2 zur Herstellung von Verbindungen der Formel IA oder IB, worin $R_1$ ein unsubstituierter oder durch Niederalkyl bis und mit 4 C-Atomen, Niederalkoxy bis und mit 4 C-Atomen oder Halogen, insbesondere Methyl, Methoxy, Fluor oder Chlor, substituierter 2- oder 4-Biphenylylrest ist, oder eines Salzes davon.

4. Verfahren nach einem der Ansprüche 1-3 zur Herstellung von Verbindungen der Formel IA oder IB, worin $R_1$ unsubstituiertes 4-Biphenylyl ist, oder eines Salzes davon.

5. Verfahren nach einem der Ansprüche 1-4 zur Herstellung von Verbindungen der Formel IA, oder eines Salzes davon.

6. Verfahren nach einem der Ansprüche 1-5 zur Herstellung von Verbindungen der Formel IA oder IB, worin X-X, Y-Y und Z-Z je Vinylen darstellen, oder eines pharmazeutisch verwendbaren Salzes davon.

7. Verfahren nach einem der Ansprüche 1-5 zur Herstellung von Verbindungen der Formel IA oder IB, worin X-X und Y-Y je Ethylen und Z-Z Vinylen bedeuten, oder eines pharmazeutisch verwendbaren Salzes davon.

8. Verfahren nach einem der Ansprüche 1-5 zur Herstellung von Verbindungen der Formel IA oder IB, worin X-X, Y-Y und Z-Z je Ethylen bedeuten, oder eines pharmazeutisch verwendbaren Salzes davon.

9. Verfahren nach Anspruch 1 zur Herstellung von 3-[4-(4-Biphenylyl-methyl)-1-piperazinyl]-rifamycin SV oder eines pharmazeutisch anwendbaren Salzes davon.

10. Verfahren nach Anspruch 1 zur Herstellung von 3-[4-(4-Biphenylyl-methyl)-1-piperazinyl]-16,17,18,19-tetrahydro-rifamycin SV oder eines pharmazeutisch anwendbaren Salzes davon.

11. Verfahren nach Anspruch 1 zur Herstellung von 3-[4-(4-Biphenylyl-methyl)-1-piperazinyl]-16,17,18,19,28,29-hexahydro-rifamycin SV oder eines pharmazeutisch anwendbaren Salzes davon.

12. Verfahren zur Herstellung von pharmazeutischen Präparaten enthaltend als Wirkstoff mindestens eine Verbindung gemäss einem der Ansprüche 1 bis 11 oder ein pharmazeutisch verwendbares Salz davon, dadurch gekennzeichnet, dass man den Wirkstoff mit einem pharmazeutischen Hilfsmaterial verarbeitet.

13. Verwendung eines Rifamycinderivats nach einem der Ansprüche 1-11 zur Herstellung von Arzneimitteln, die für die Anwendung zur Behandlung bakterieller Erkrankungen bestimmt sind und die für die Anwendung zur Behandlung viraler Erkrankungen durch Typ C-Retroviren bestimmt sind.

Europäisches Patentamt

# EUROPÄISCHER RECHERCHENBERICHT

Nummer der Anmeldung

EP 88810533.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | WO - A1 - 87/02 361 (CIBA-GEIGY)<br>* Beispiele; Ansprüche; Seite 13 *<br>-- | 1-16 | C 07 D 498/08<br>A 61 K 31/395 |
| A | US - A - 4 353 826 (BICKEL et al.)<br>* Zusammenfassung; Beispiel 77 *<br>-- | 1-14, 16 | |
| D,A | US - A - 4 005 077 (BICKEL et al.)<br>* Beispiel 77; Ansprüche, Kolonne 6, Zeilen 1-19 *<br>-- | 1-14, 16 | |
| A | DE - A1 - 2 816 274 (DSO, PHARMACHIM)<br>* Ansprüche; Tabelle 1 *<br>---- | 1-14, 16 | |

| RECHERCHIERTE SACHGEBIETE (Int. Cl.4) |
|---|
| C 07 D 498/00 |

Der vorliegende Recherchenbericht wurde für alle Patentansprüche erstellt.

| Recherchenort | Abschlußdatum der Recherche | Prüfer |
|---|---|---|
| WIEN | 17-11-1988 | JANISCH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument